Europäisches Patentamt

European Patent Office

Office européen des brevets

(19)

(11) Publication number: **0 392 820**
**A2**

(12) **EUROPEAN PATENT APPLICATION**

(21) Application number: **90303907.1**

(22) Date of filing: **11.04.90**

(51) Int. Cl.5: **C12N 15/30, C12P 21/02,**
**A61K 39/015**

(30) Priority: **11.04.89 US 336288**

(43) Date of publication of application:
**17.10.90 Bulletin 90/42**

(84) Designated Contracting States:
**GR**

(71) Applicant: **CHIRON CORPORATION**
**4560 Horton Street**
**Emeryville California 94608(US)**

(72) Inventor: **Barr, Philip J.**
**67 Bay Forest Drive**
**Oakland, California 94611(US)**
Inventor: **Bathurst, Ian C.**
**851 Solano Avenue**
**Albany, California 94706(US)**
Inventor: **Gibson, Helen L.**
**5351 Broadway**
**Oakland, California 94618(US)**

(74) Representative: **Goldin, Douglas Michael et al**
**J.A. KEMP & CO. 14, South Square Gray's Inn**
**London WC1R 5EU(GB)**

(54) **Plasmodium circumsporozoite protein analogs lacking repeat sequences.**

(57) Recombinant immunogenic analogs of Plasmodium circumsporozoite (CS) proteins useful as malaria subunit vaccines which lack one or more of the repeat epitopes of the native CS protein but have at least one nonrepeat flanking epitope from each of the amino terminal and carboxy terminal flanking regions of the native CS protein.

EP 0 392 820 A2

## PLASMODIUM CIRCUMSPOROZOITE PROTEIN ANALOGS LACKING REPEAT SEQUENCES

### Technical Field

This invention is in the fields of molecular biology and biochemistry. More particularly it relates to immunogenic analogs of Plasmodium circumsporozoite (CS) proteins that lack one or more of the repeat sequences of the native proteins.

### Background

Malaria is a debilitating disease caused by infection by a parasite of the genus Plasmodium. It is transmitted through mosquito bites. As the mosquito feeds, sporozoites, which are the differentiated form of the parasite found in the salivary gland, enter the host's bloodstream. The sporozoites ultimately enter the primary target cells, i.e., liver cells. The major Plasmodium species infective to humans are P. falciparum and P. vivax. Other species, such as P. ovale and P. malarie infect humans with much less frequency. Still other species of the parasite infect other mammals, such as P. berghei which infects rodents.

A substantial amount of prior effort has gone into attempts to make subunit vaccines against malaria. A significant portion of this work has been done by workers at New York University (NYU) and has focused on the CS protein which covers the surface of the sporozoite. NYU has filed a series of patent applications on CS protein technology.

Their first patent, U.S. 4,446,917, describes a class of sporozoite proteins designated P-44 (the P signifying Plasmodium, and the 44 signifying their approximate molecular weight in kilodaltons) that were purified from sporozoite lysates.

WO 84/02922 describes the identification of an immunodominant region of the CS protein consisting of tandem repeats of a relatively short sequential epitope and the synthesis of peptides composed of repeats of such epitopes. WO 86/00911 describes the identification and characterization of the repeat sequential epitope of P. falciparum CS protein and the synthesis of peptides composed of tandem repeats thereof. Similarly WO 87/00533 describes the identification and characterization of the repeat sequential epitope of P. vivax CS protein. While that patent application indicates that other segments of the CS protein may be included with the tandem repeats, the clear teaching of the reference is that the immunogenicity of the protein is attributable to the presence of the repeated epitope. No example of any peptide that includes flanking regions is given.

WO 86/01721 teaches that certain charged regions on either side of the tandem repeat epitope of the CS protein are immunogenic. Peptides corresponding in sequence to those regions were synthesized and shown to bind to anti-CS antibodies. Conjugates of those synthetic peptides were reported to induce anti-CS antibodies in test animals.

U.S. Patent Application Serial No. 032327, which is co-owned by NYU and the assignee of the present invention, describes an immunogenic P. vivax CS protein analog composed of the entire tandem repeat region of the native molecule and selected portions of the flanking sequences on either side of the repeat sequence. This polypeptide has exhibited superior immunogenic properties than the peptides that were prepared by the applicants of the above described NYU patents/applications.

Eichinger, et al., Molecular and Cellular Biology (1986) 6:3965-3972, de la Cruz, et al., Molecular and Biochemical Parasitology (1988) 28:31-38, and Romero, et al., Vaccines 89 (1989) Cold Spring Harbor, pp. 335-340, characterize the CS protein of P. berghei and the gene encoding it.

Despite all of this prior literature on Plasmodium CS proteins it is still very unclear which constructs will provide in vivo responses in humans that protect against malaria. In this regard the role of the immune response to regions other than the tandem repeats in sporozoite neutralization has not been extensively assessed because of the extreme immunodominance of the repeat region.

### Disclosure of the Invention

The present invention is based on the concept that reduction of the immunodominance of the repeat region relative to the epitopes in the regions flanking the repeat region will qualitatively enhance sporozoite neutralizing activity.

Accordingly, the invention is directed to immunogenic Plasmodium CS protein analogs that lack one or more of the repeat epitopes of the native CS protein but have at least one nonrepeat flanking epitope from each nonrepeat flanking region of the native CS protein, recombinant polynucleotides and transformed hosts for making such proteins, and vaccine compositions that employ such proteins as an immunogen.

More specifically, one aspect of the invention is an immunogenic Plasmodium CS protein analog comprising:

(a) an internal region or domain composed of at least two iterations of a repeat sequence of a native Plasmodium CS protein, but fewer iterations of said repeat sequence than are present in the native Plasmodium CS protein;

(b) an amino terminal region or domain flanking the internal region having an amino acid sequence that defines at least one epitope of the nonrepeat amino flanking region of the native Plasmodium CS protein; and

(c) a carboxy terminal region or domain flanking the internal region having an amino acid sequence that defines at least one epitope of the nonrepeat carboxy flanking region of the native Plasmodium CS protein.

Other aspects of the invention are recombinant polynucleotides that encode the above-described Plasmodium CS protein analogs, expression vectors for expressing such polynucleotides in a host cell comprising such DNA operably connected to DNA that enables the expression of the DNA in the host cell, and host cells that are transformed with such vectors and are capable of producing such analogs.

Still other aspects of the invention are vaccines that contain the immunogenic CS protein analogs and methods for protecting individuals against malaria by immunizing them with such vaccines.

Brief Description of the Drawings

In the drawings:

Figure 1 shows the amino acid sequence of and a DNA sequence encoding the P. vivax CS protein analog Vivax 3.

Figure 2 shows the oligomers and ligation scheme that were used to construct the DNA sequence shown in Fig. 1.

Figure 3 is a schematic diagram of plasmid pBS100.

Figure 4 is a schematic diagram of plasmid pBS24.1

Figure 5 shows the amino acid sequence of and a DNA sequence encoding the P. vivax CS protein analog Vivax 3.1.

Figure 6 shows the oligomers and ligation scheme that were used to construct the DNA sequence shown in Figure 5.

Figure 7 is a schematic diagram of plasmid pAB125.

Figure 8 shows the amino acid sequence of and a DNA sequence encoding the P. falciparum CS protein analog Falciparum 3.

Figure 9 shows the oligomers and ligation scheme that were used to construct the DNA sequence shown in Figure 8.

Figure 10 shows the amino acid sequence of and a DNA sequence encoding the P. falciparum CS protein analog Falciparum 4.

Figure 11 shows the amino acid sequence of and a Dna sequence encoding the P. berghei CS protein analog Berghei 3.

Figure 12 shows the oligomers and ligation scheme used to construct the DNA sequence of Figure 11.

Modes for Carrying Out the Invention

1. Definitions

The term "repeat sequence of a native Plasmodium CS protein" intends the repeated amino acid sequences that appear in the central immunodominant regions or domains of native Plasmodium CS proteins and modifications of those repeat sequences that do not destroy their immunogenic properties. The native repeat sequences for P. vivax and P. falciparum are described in the NYU patent applications cited above. The native repeat sequences for P. berghei are described by Eichinger, et al., supra. For example, the repeat sequences of P. vivax are Asp-Arg-Ala-Asp/Ala- Gly-Glu-Pro-Ala-Gly, those of P. falciparum are Asn-Ala-Asn-Pro and Asn-Val-Asp-Pro, and those of P. berghei are Pro-Pro/Ala-Pro-Pro-Asn-Pro/Ala-Asn-Asp.

The term "nonrepeat flanking amino region of a native Plasmodium CS protein" intends the portion of that protein that extends from the amino terminal of the protein to the repeat sequence region of the protein. Correspondingly, the term "nonrepeat flanking carboxy region of a native Plasmodium CS protein" means the portion of that protein that extends from the carboxy terminal of the protein to the repeat sequence region.

The term "internal" as used in describing an amino acid sequence of a protein intends a sequence that does not include a terminal amino acid of the protein.

"Host cells", "cells", "cell lines", "cell cultures", and other such terms denoting prokaryotic microorganisms or eukaryotic cell lines cultured as unicellular entities, are used interchangeably, and refer to cells which can be, or have been, used as recipients for recombinant vectors or other transfer DNA, and include the progeny of the original cell which has been transfected. It is understood that the progeny of a single parental cell may not necessarily be completely identical in morphology or in genomic or total DNA complement as the original parent, due to accidental or deliberate mutation. Progeny of the parental cell which are sufficiently similar to the parent to be characterized by the relevant property, such as the presence of a nucleotide sequence encoding a desired peptide, are included in the progeny intended by this definition, and are covered by the above terms.

A "replicon" is any genetic element, e.g., a plasmid, a chromosome, a virus, that behaves as an autonomous unit of polynucleotide replication within a cell.

A "vector" is a replicon in which another polynucleotide segment is attached, so as to bring about the replication and/or expression of the attached segment.

"Control sequence" refers to polynucleotide sequences which are necessary to effect the expression of coding sequences to which they are ligated. The nature of such control sequences differs depending upon the host organism; in prokaryotes, such control sequences generally include promoter, ribosomal binding site, and terminators; in eukaryotes, generally, such control sequences include promoters, terminators and, in some instances, enhancers. The term "control sequences" is intended to include, at a minimum, all components whose presence is necessary for expression, and may also include additional components whose presence is advantageous, for example, leader sequences.

"Operably connected" refers to a juxtaposition wherein the components so described are in a relationship permitting them to function in their intended manner. A control sequence "operably connected" to a coding sequence is ligated in such a way that expression of the coding sequence is achieved under conditions compatible with the control sequences.

The term "immunogenic" as used to describe the invention analogs intends the ability to cause Plasmodium sporozoite neutralization by inducing a humoral and/or cellular response. The term "neutralization" intends a state incapable of causing infection and/or malarial symptoms.

"Transformation", as used herein, refers to the insertion of an exogenous polynucleotide into a host cell, irrespective of the method used for the insertion, for example, direct uptake, transduction, or f-mating. The exogenous polynucleotide may be maintained as a non-integrated vector, for example, a plasmid, or alternatively, may be integrated into the host genome.

"Treatment" as used herein refers to prophylaxis and/or therapy.

An "individual", as used herein refers to an animal that is susceptible to infection by one or more specie of Plasmodium and includes, but is not limited to, primates and humans.

## 1. Characterization of CS Protein Analogs

As indicated above the features that distinguish the invention analogs from previous CS protein analogs are that the former lack one or more repeat sequences found in the native protein but retain sequential epitopes from both flanking regions of the native protein.

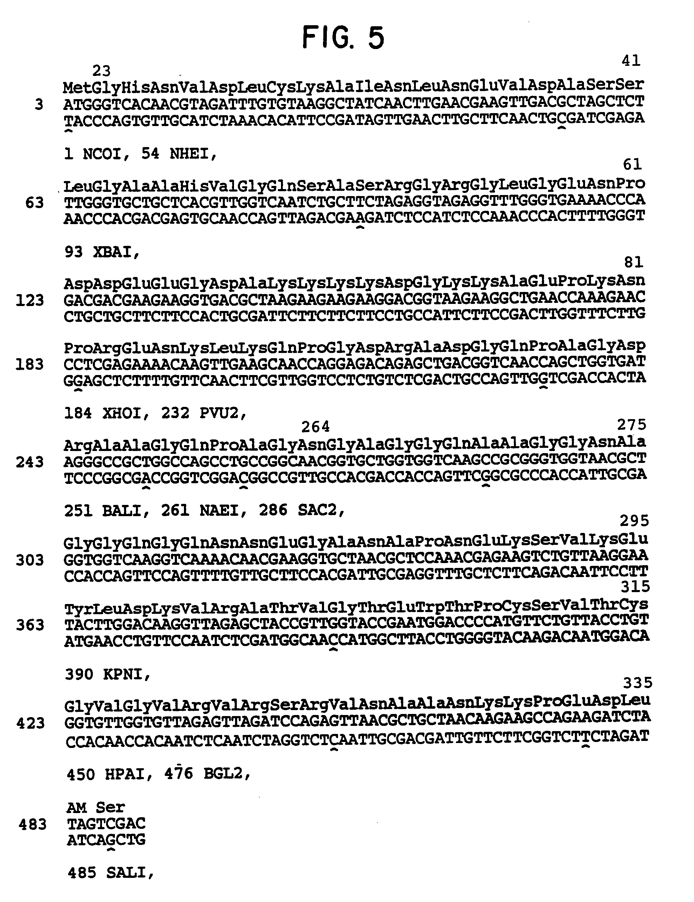

The native P. vivax CS protein consists of 373 amino acids. It has an N-terminal hydrophobic leader sequence extending from amino acids 1-22, (all amino acid ranges herein are inclusive, numbering of amino acids in the native P. vivax CS protein is according to that shown in the Vivax 3.1 sequence of Figure 5). The tandem repeats, of which there are 19, begin at amino acid 92 and extend through amino acid 263 and are preceded by a conserved region, called Region I, that extends from amino acid 77 through amino acid 91. The C-terminal region ends with a transmembrane sequence extending from amino acid 356 through amino acid 373 and also contains a conserved region, called Region II, at amino acids 306 through 318. Nine of the repeat sequences are Asp-Arg-Ala-Asp-Gly-Gln-Pro-Ala-Gly (designated sequence "A") and ten are Asp-Arg-Ala-Ala-Gly-Gln-Pro-Ala-Gly (designated sequence "B").

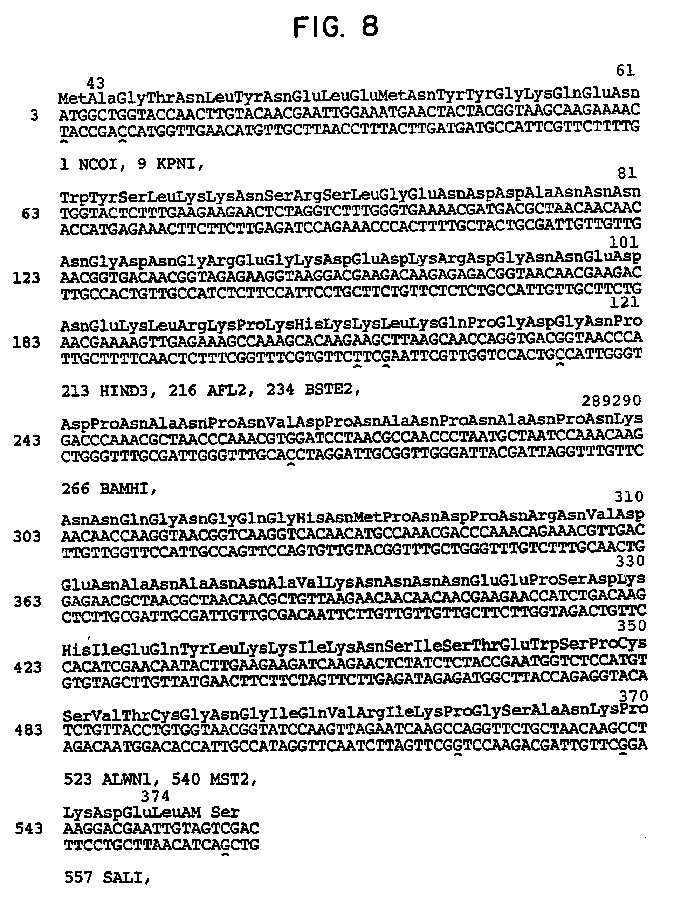

Correspondingly, the P. falciparum CS protein consists of 412 amino acids. Its N-terminal hydrophobic leader sequence extends from amino acid 1 through amino acid 42 and its Region I is located at amino acids 107-123, inclusive. (Numbering of amino acids in the P. falciparum CS protein is according to that shown in the Falc3 sequence shown in Figure 8.) There are 41 repeats in the protein, with four being Asn-Val-Asp-Pro (Sequence "A") and the remainder being Asn-Ala-Asn-Pro (Sequence "B"). Region II of the P. falciparum CS protein is located at amino acids 344-356, and its transmembrane sequence is at amino acids 391-412.

The native P. berghei CS protein as reported by Eichinger et al., supra, (as corrected by de la Cruz et al., supra) consists of 339 amino acids. The tandem repeats, of which there are 13, begin at amino acid 93 and end at amino acid 196. Five of the repeat sequences are Pro-Pro-Pro-Pro-Asn-Pro-Asn-Asp (designated sequence "A"), two are Pro-Pro-Pro-Pro-Asn-Ala-Asn-Asp, and six are Pro-Ala-Pro-Pro-Asn-Ala-Asn-Asp (designated sequence B). Region I extends from approximately amino acid 82 through amino acid 92. Region II extends from amino acid 272 through amino acid 286. The N-terminal hydrophobic leader comprises amino acids 1 through 23.

The analogs of this invention contain at least two repeat sequences of a native CS protein, preferably with both types of repeat sequence being present. To the extent possible, the ratios of sequence A to sequence B are preserved in the analog and the distribution (i.e., respective positioning of the two types of repeats) are also preserved.

In this regard, the A sequences of P. vivax are predominant at the N-terminal end of the series of tandem repeats. The same applies in the cases of the P. falciparum and P. berghei proteins. Accordingly, in the P. vivax analogs of the invention there are 2-18 repeats, preferably 2 to 10 repeats, with an approximately equal number of sequence A and sequence B repeats with the A sequence(s) located predominantly in the N-terminal portion of the series. Correspondingly, the P. falciparum analogs will contain 2 to 40 repeats, preferably 2 to 20 repeats, with an A-to-B ratio of about 1:10 to 3:10. The P. berghei analogs will contain 2 to 12 repeats.

The N-terminal flanking region will typically comprise Region I and optionally additional flanking sequence out to the hydrophobic leader. Thus, the N-terminal flanking region of the P. vivax analogs will typically contain at least amino acids 77-91 of the native P. vivax CS protein and optionally all or a portion of amino acids 23-76. Preferably it consists of amino acids 23-91. Correspondingly the N-terminal flanking region of the P. falciparum analogs will contain at least amino acids 107-123 of the native P. falciparum CS protein and optionally all or a portion of amino acids 43-106. Preferably it consists of amino acids 43-123. In the case of the P. berghei analogs the N-terminal flanking region will typically contain at least amino acids 82 to 92.

The C-terminal flanking region will typically comprise the native sequence extending from the end of the repeat region through Region II and optionally all ·or a part of the sequence extending from the end of Region II to the transmembrane sequence. Thus, the C-terminal flanking region of the P. vivax analogs will typically contain at least amino acids 264-318 of the native protein and optionally all or a portion of amino acids 319-355. Preferably it consists of amino acids 264-335 of the native protein. The corresponding C-terminal region of the P. falciparum analogs will contain at least amino acids 289-356 and optionally all or a portion of amino acids 357-392.

The N-terminal and C-terminal regions of the analogs may vary in amino acid sequence from corresponding native CS sequences or fragments thereof due to one or more substitutions, additions, and/or deletions (a "deletion" refers to elimination of internal residuals rather than shortening a terminus) provided, however, that they each continue to define at least one epitope. Normally there will not be more than about 10 variations per region, preferably not more than about 5 variations per region.

The P. vivax, P. falciparum, and P. berghei CS protein analogs of the invention may be represented by the following formula:

X-(A$_n$B$_m$)-Y

where (A$_n$B$_m$) represents an internal tandem repeat region, A represents one of the repeat sequences, A represents one of the repeat sequences of the native CS protein, B represents another of the repeat sequences, n and m are positive integers, the sum of n and m is less than the number of repeat sequences in the native CS protein, X represents an N-terminal flanking sequence and defines at least one sequential epitope of the N-flanking region of the native CS protein, and Y represents a C-terminal flanking sequence and defines at least one sequential epitope of the C flanking region of the native CS protein. In the case of P. vivax analogs, X represents at least amino acids 77-91 of the native protein and Y represents at least amino acids 264-318 of the native protein, whereas in the P. falciparum analogs, X represents at least amino acids 107-123 of the native protein and Y represents at least amino acids 289-356 of the native protein.

## Recombinant Materials and Methods for Producing CS Protein Analogs

The CS protein analogs of the invention are made by recombinant DNA techniques. Based on the known amino acid sequences for the native repeat sequences and flanking sequences, synthetic genes encoding the analogs may be prepared in vitro by synthesizing appropriate oligonucleotide fragments of the gene and kinasing and ligating them together. For expression in a particular host, it may be desirable to design a synthetic DNA sequence that employs codons preferred by the particular host in which the DNA is to be expressed.

The synthetic DNA encoding the analogs may be cloned into any suitable replicon to create a cloning vector. Numerous cloning vectors are known to those of skill in the art, and the selection of an appropriate cloning vector is a matter of choice. Examples of vectors for cloning and host cells which they can transform include the bacteriophage lambda (E. coli), pBR322 (E. coli), pACYC177 (E. coli), pKT230 (gram-negative bacteria), pME290 (non-E. coli gram-negative bacteria), pHV14 (E. coli and Bacillus subtilis), pBD9 (Bacillus), plJ61 (Streptomyces), pUC6 (Streptomyces)1 actinophage phiC31 (Streptomyces), Ylp5 (Saccharomyces), YCp19 (Saccharomyces), YEp24 and YEp13 (Saccharomyces), and bovine papilloma virus (mammalian cells).

The polynucleotide sequences encoding the CS protein analogs are expressed by inserting the sequences into appropriate replicons, thereby creating expression vectors, and introducing the resulting expression vectors into compatible hosts.

In creating an expression vector, the sequence encoding the CS protein analog is located in the vector with the appropriate control sequences. The positioning and orientation of the coding sequence with respect to the control sequences is such that the coding sequence is transcribed under the control of the control sequences: i.e., the promoter will control the transcription of the mRNA derived from the coding sequence, and the ribosomes will bind at the ribosomal binding site to begin the translational process, and the stop codon used to terminate translation will be upstream from the transcriptional termination codon. Yeast expression vectors are preferred. Control sequences for yeast vectors include promoters for the synthesis of glycolytic enzymes (Hess et al., J Adv Enzyme Reg (1968) 7:149; Holland et al., Bio chemistry (1978) 17:4900). Additional promoters known in the art include the promoter for 3-phosphoglycerate kinase (Hitzeman et al., J Biol Chem (1980) 255:2073). Other promoters, which have the additional advantage of transcription controlled by growth conditions and/or genetic background are the promoter regions for alcohol dehydrogenase 2 (ADH2), isocytochrome C, acid phosphatase, degradative enzymes associated with nitrogen metabolism, the alpha factor system, and enzymes responsible for maltose and galactose utilization. Transcription terminator sequences are desirable at the 3′ end of the coding sequences. Such terminators are found in the 3′ untranslated region following the coding sequences in yeast-derived genes.

Modification of the sequence encoding the analog, prior to its insertion into the replicon, may be desirable or necessary, depending upon the expression system chosen. For example, in some cases, it may be necessary to modify the sequence so that it may be attached to the control sequences with the appropriate orientation, i.e., to maintain the reading frame. In some cases it may be desirable to add sequences such as yeast alpha factor leader sequences, which cause the secretion of the polypeptide from the host organism, with subsequent cleavage of the secretory signal. The techniques for modifying nucleotide sequences utilizing cloning are well know to those skilled in the art. They include, e.g., the use of restriction enzymes, enzymes such as Bal31, to remove excess nucleotides, chemically synthesized oligonucleotides for use as adapters to replace lost nucleotides.

The sequence encoding the analog, modified if necessary, may be ligated to the control sequences prior to insertion into a vector. Alternatively, the coding sequence can be cloned directly into an expression vector which already contains the control sequences and an ap propriate′ restriction site. For expression of the analog in yeast, the control sequences will necessarily be heterologous to the coding sequence. In cases where the analog gene is to be expressed in cell lines derived from vertebrates, the control sequences may be either heterologous or homologous, depending upon the particular cell line.

Depending on the host cell used, transformation is done using standard techniques appropriate to such cells. Transformations into yeast may be carried out according to the method of Beggs, J.D., Nature (1978) 275:104-109, or of Hinnen, A., et al., Proc Natl Acad Sci (USA) (1978) 75:1929.

Transformed cells are then grown under conditions which permit expression of the CS protein analog gene and, if appropriate, processing into the mature protein. Because the gene is expressed in heterologous organisms/cells, the protein is free of the other proteins with which it is associated in mosquitoes. The thus synthesized recombinant CS protein analog is then isolated from the host cells and purified. If the expression system secretes the analog into the growth media, the analog is isolated directly from the media. If the recombinant analog is not secreted, it is isolated from cell lysates. The selection of the appropriate growth conditions and recovery methods are within the skill of the art. With regard to purification, see, for instance, the NYU patent applications described above.

## Vaccines

Vaccines may be prepared from the recombinant Plasmodium CS protein analogs described above which have sporozoite neutralizing activity or ameliorate or prevent malaria by other mechanisms. The formulation of vaccines which contain immunogenic polypeptide(s) as active ingredients is known to those skilled in the art. Such vaccines are typically prepared as injectables, either as liquid solutions or suspensions; solid forms suitable for solution in, or suspension in, liquid prior to injection may also be prepared. The preparation may also be emulsified, or the protein encapsulated in liposomes. The active immunogenic ingredients are often mixed with excipients or carriers which are pharmaceutically acceptable and compatible with the active ingredient. Suitable excipients are, for example, water, saline, dextrose, glycerol, ethanol, or the like and combinations thereof. The concentration of analog in injectable formulations will usually be in the range of 0.2 to 5 mg/ml. In addition, if desired, the vaccine may contain minor amounts of auxiliary substances such as wetting or emulsifying agents, pH buffering agents, and/or adjuvants which enhance the effectiveness of the vaccine. Examples of adjuvants which may be effective include but are not limited to: aluminum hydroxide, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxyphosphoryloxy)ethylamine (CGP 19835A, referred to as MTP-PE), and RIBI, which contains three components extracted from bacteria, monophosphoryl lipid A, trehalose dimycolate and cell wall skeleton (MPL + TDM + CWS) in a 2% squalene/Tween 80 emulsion. The effectiveness of an adjuvant may be determined by measuring the amount of antibodies directed against the immunogen resulting from administration of the immunogen in vaccines which are also comprised of the various adjuvants.

The vaccines are conventionally administered parenterally, by injection, for example, either intravenously, subcutaneously or intramuscularly. Additional formulations which are suitable for other modes of administration include suppositories and, in some cases, oral formulations and liposomes. For suppositories, traditional binders and carriers may include, for example, polyalkalyne glycols or triglycerides; such suppositories may be formed from mixtures containing the analog in the range of 0.5% to 10% by weight, preferably 1%-2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10%-95% of analog, preferably 25%-70%.

The analogs may be formulated into the vaccine as neutral or salt forms. Pharmaceutically acceptable salts include the acid addition salts (formed with free amino groups of the peptide) and which are formed with inorganic acids such as, for example, hydrochloric or phosphoric acids, or such organic acids such as acetic, oxalic, tartaric, maleic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The vaccines are administered in a manner compatible with the dosage formulation, and in such amount as will be prophylactically and/or therapeutically effective. The quantity to be administered, which is generally in the range of 5 micrograms to 250 micrograms of analog per dose, depends on the subject to be treated, capacity of the individual's immune system to synthesize antibodies, and the degree of protection desired. Precise amounts of active ingredient required to be administered may depend on the judgment of the physician and may be peculiar to each individual.

The vaccine may be given in a single dose schedule, or preferably in a multiple dose schedule. A multiple dose schedule is one in which a primary course of vaccination may be with 1-10 separate doses, followed by other doses given at subsequent time intervals required to maintain and or re-enforce the immune response, for example, at 1-4 months for a second dose, and if needed, a subsequent dose(s) after several months.

## Examples

The following examples further illustrate the invention analogs and method for their preparation. They are not intended to limit the invention in any manner.

## Vivax 3 Expression Vector

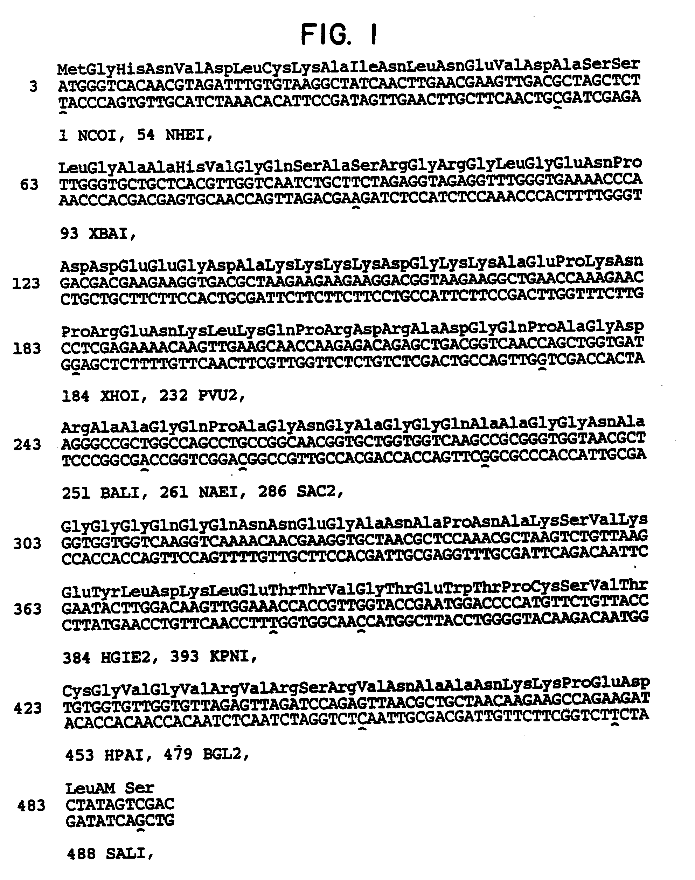

Vivax 3 is a P. vivax CS protein analog consisting of amino acids 23-91 of native P. vivax CS protein (with a Gly-->Arg substitution at position 91), followed by a single "A" sequence (Asp-Arg-Ala-Asp-Gly-Gln-Pro-Ala-Gly), followed by a single "B" sequence (Asp-Arg-Ala-Ala-Gly-Gln-Pro-Ala-Gly), followed by amino acids 264-335 (with an added Gly between positions 277-278, a Glu-->Ala substitution at 290, a Val-->Leu substitution at 300, an Arg-Glu substitution at 301 and an Ala-->Thr substitution at 302) of the native P. vivax CS protein. The amino acid sequence and DNA encoding sequence for Vivax 3 are shown in Figure 1.

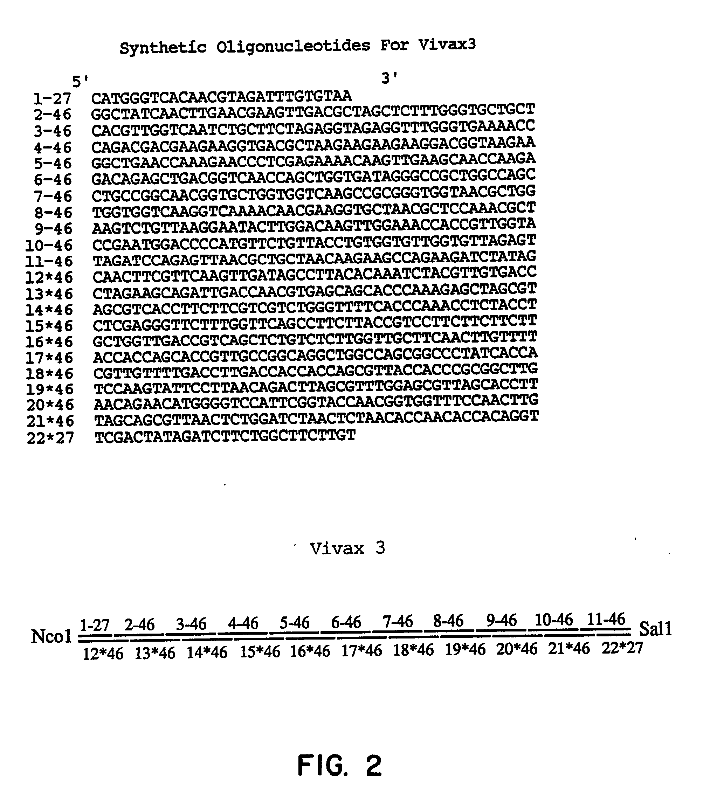

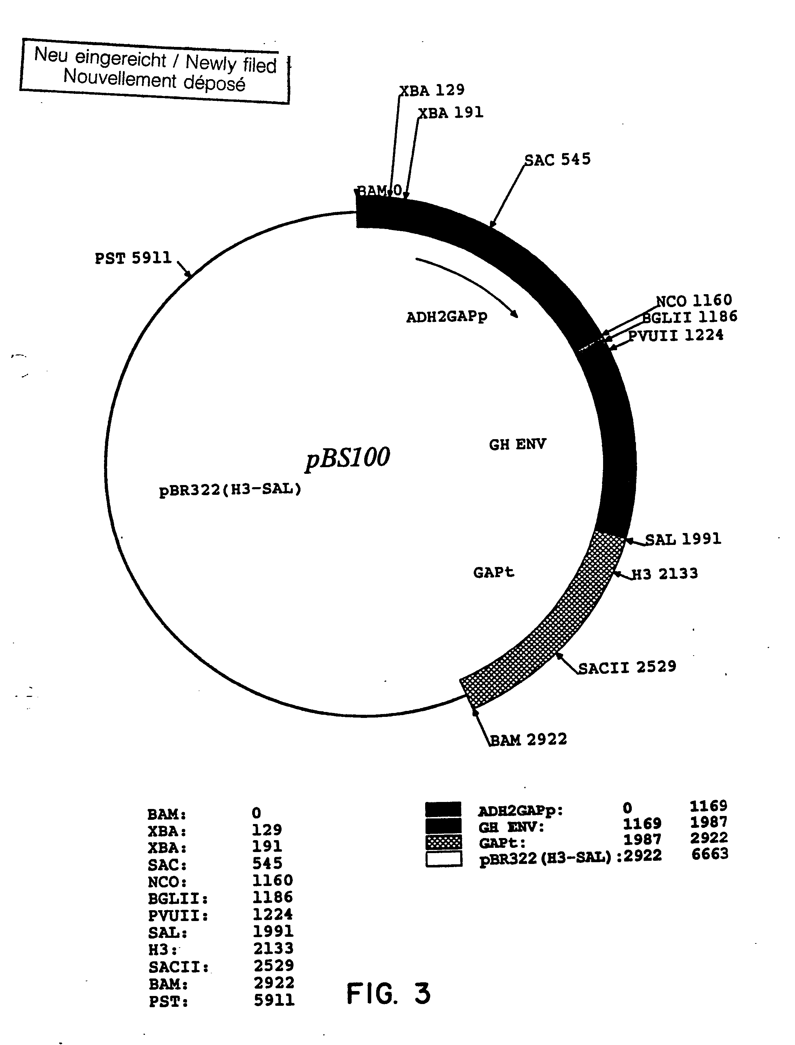

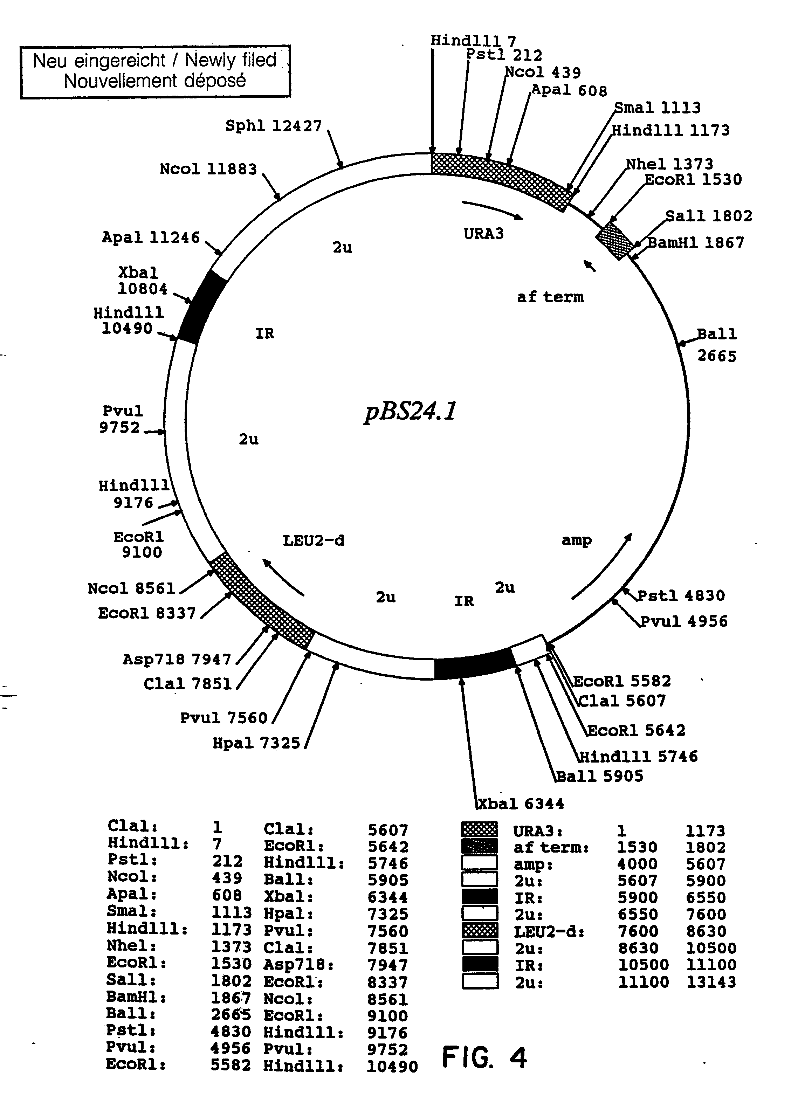

Twenty-two synthetic oligomers, shown in Figure 2, were made on an Applied Biosystem 380A DNA synthetizer. These oligonucleotides were kinased and annealed by boiling and cooling slowly to room temperature and then ligated to foam a DNA sequence encoding Vivax 3 (see scheme in Figure 2). The full-size gene for Vivax 3 was excised from a 7% acrylamide gel and cloned into plasmid pBS100 that had been digested with Ncol and Sall. pBS100 is schematically depicted in Figure 3 and contains the in- frame ADH2-GAPDH hybrid promoter and GAPDH terminator. A BamHI/Sall fragment containing the promoter, DNA sequence encoding Vivax 3, and the terminator was isolated from the cloning vector and inserted into plasmid pBS24.1 to yield expression vector pBS24vivax3 for intracellular expression in yeast. pBS24.1 is schematically depicted in Figure 4.

## Vivax 3.1 Expression Vector

Vivax 3.1 is a P. vivax CS protein analog that is the same as Vivax 3 except that it does not have the noted amino acid variations in the flanking regions from the native sequence. The amino acid sequence and DNA encoding sequence for Vivax 3.1 are shown in Figure 5.

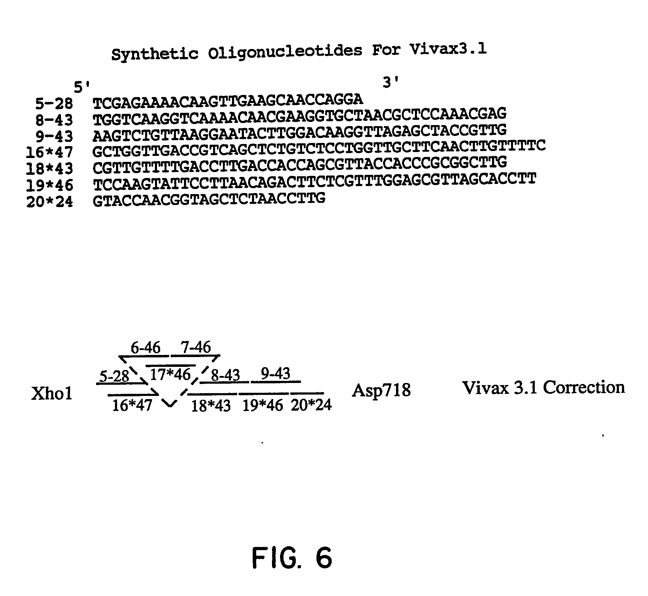

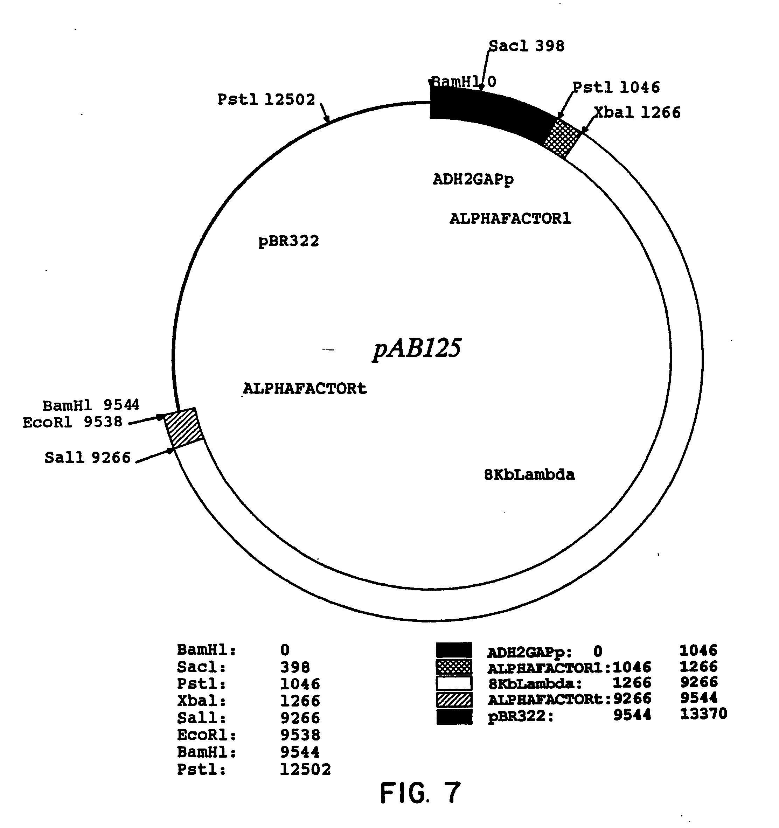

A gene encoding Vivax 3.1 was prepared as above using the oligomers and ligation scheme shown in Figure 6. This gene was cloned into plasmid pAB125 that had been digested with Xbai and SalI. pAB125 is schematically depicted in Figure 7 and contains the in-frame ADH2-GAPDH hybrid promoter, S. cerevisiae alpha factor leader and alpha factor terminator. A BamHI/SalI fragment containing the promoter, alpha factor leader, Vivax 3.1 gene, and terminator was isolated from the cloning vector and inserted into plasmid pBS24.1 to yield expression vector pBS24vivax3.1 for secretory expression in yeast.

Falciparum 3 Expression Vector

Falciparum (Falc) 3 is a P. falciparum CS protein analog consisting of amino acids 43-123 of the native P. falciparum CS protein, followed by four repeat sequences (three "B"s, i.e., -Asn-Ala-Asn-Pro- and one A, i.e., Asn-Val-Asp-Pro in a BABB pattern), followed by amino acids 289-374 of the native protein. The amino acid sequence and DNA encoding sequence for Falc 3 are shown in Figure 8.

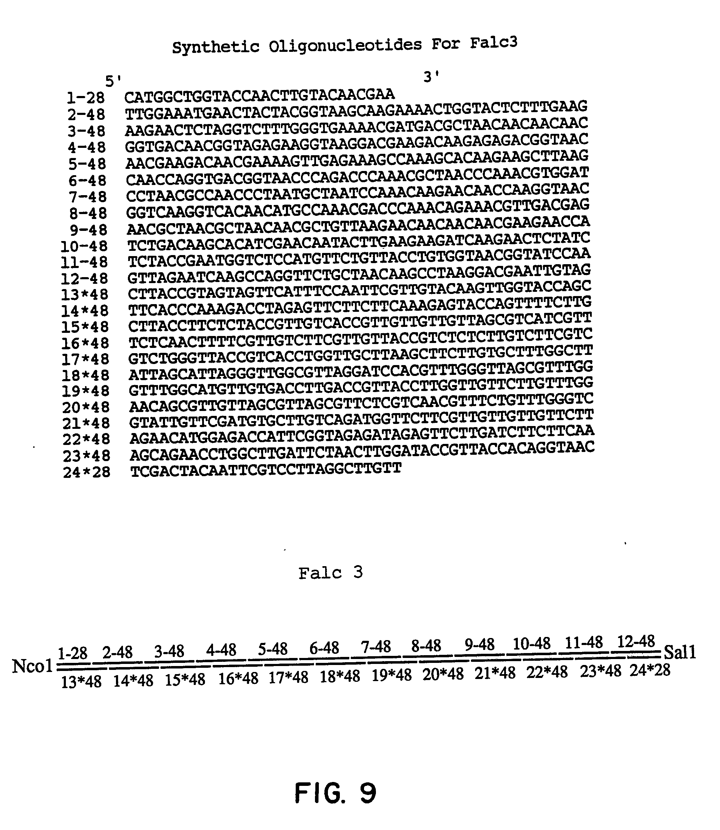

A gene encoding Falc 3 was synthesized from oligomers (see Figure 9), cloned into pAB125 and the resulting ADH2/GAPDH-alpha factor-Falc 3 construct cloned into pBS24.1 to yield expression vector pBS24falc3 for secretory expression of Falc 3 in yeast.

Falciparum 4 Expression Vector

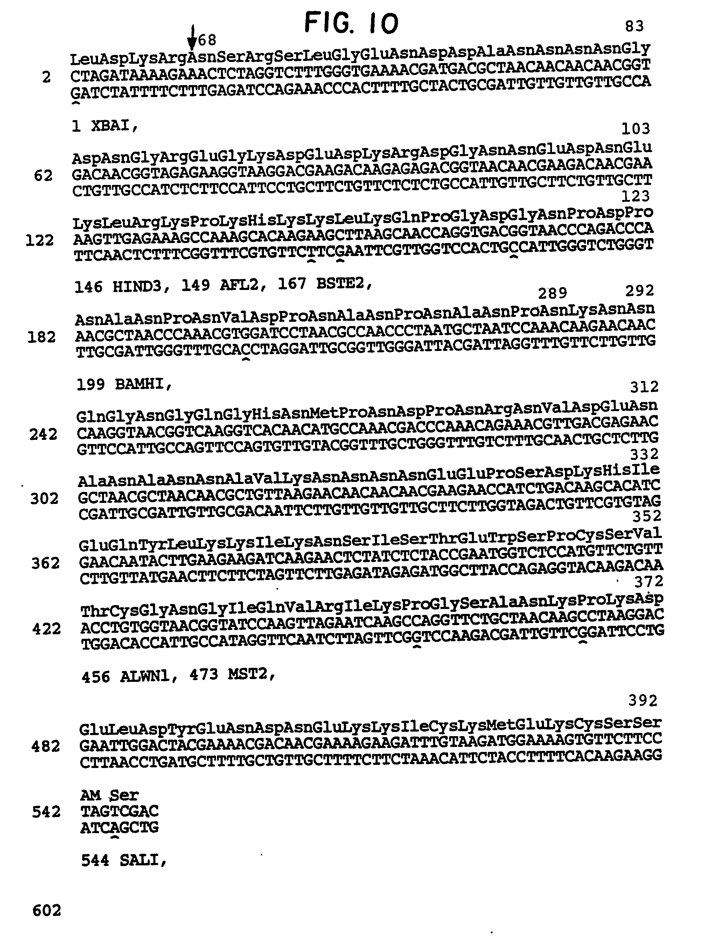

Falc 4 is a CS protein analog having a sequence similar to Falc 3 except that it has a shorter amino flanking region and a longer carboxy flanking region than Falc 3. The amino acid sequence and DNA sequence encoding Falc 4 are shown in Fig. 10. As indicated, the seqeunce includes amino acids 68-123 of the native P. falciparum CS protein sequence, four repeats, and amino acids 289-392 of the native sequence. The additional amino acids at the, N-terminus of the sequence are part of the alpha factor leader sequence.

The DNA encoding the Falc 4 sequence shown in Fig. 10 was synthesized and cloned into pAB125 and pBS24.1 as above to provide expression vector pBS24falc4 for secretory expression in yeast.

Berghei 3 Expression Vector

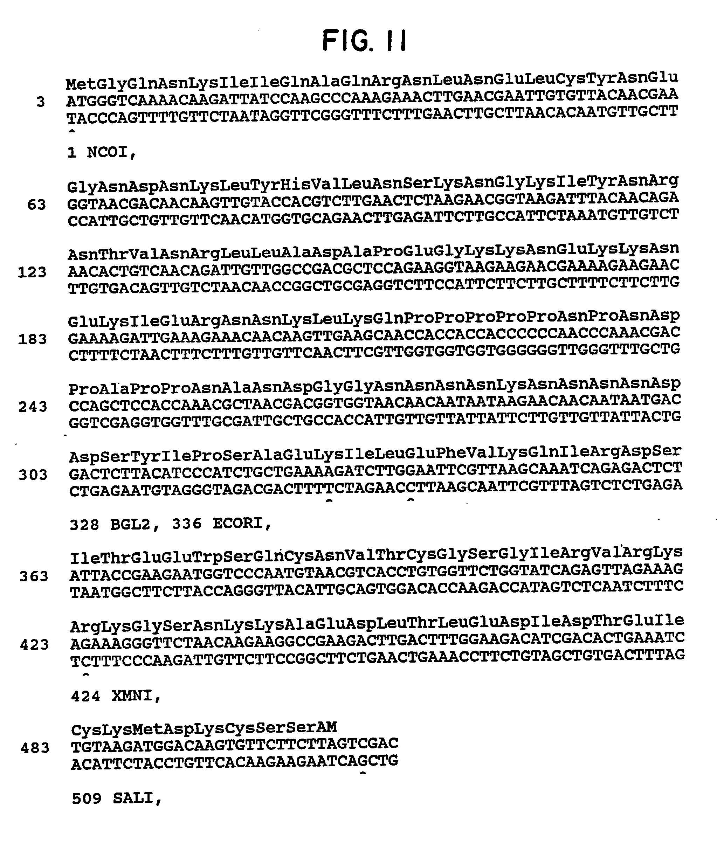

Berghei 3 is a synthetic gene containing amino and carboxy terminal flanking regions and only 2 of the 13 eight amino acid tandem repeats found in the P. berghei CS gene. The 16 QP repeats following the amino acid repeats are also eliminated. The sequence for the gene and the amino acid encoded thereby are shown in Figure 11.

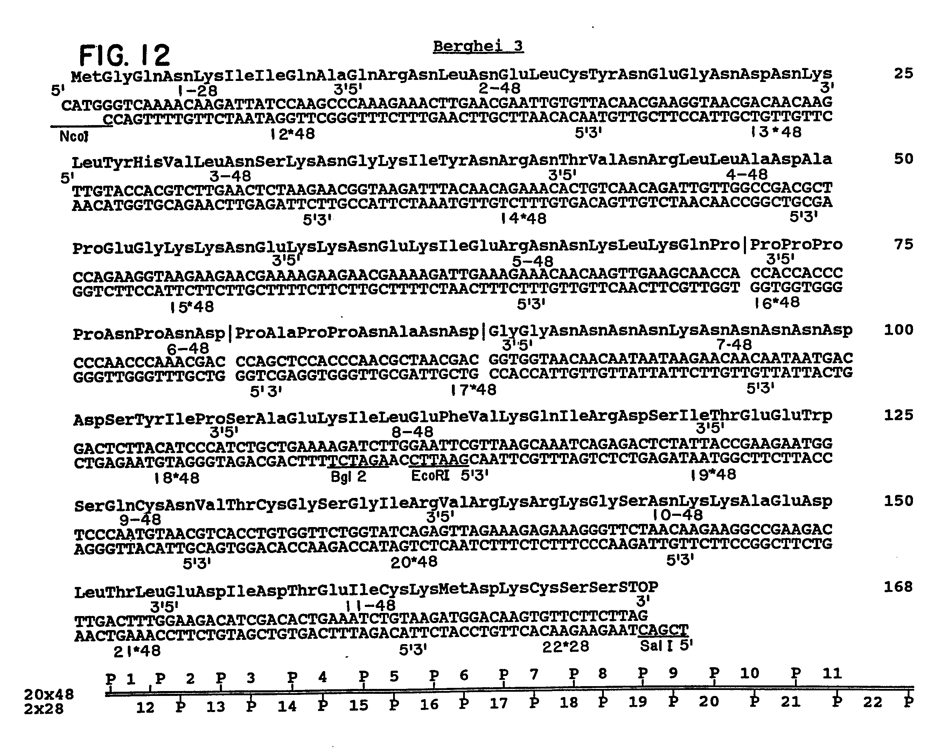

Thus, the construct begins on Gly 63 and continues through the first PPPPNPND repeat ending on amino acid 100. Note that amino acids 69-74 originally published by Eichinger et al. as PMLRRK have been changed to the "corrected" sequence of de la Cruz et al. to ADAPEG. The second repeat PAPPNAND is a derivative of the first found further into the sequence. The carboxy terminus then continues at Gly 239 through Ser 318. As shown in Figure 12, the gene was made with 20 48-mers and 2 28-mers to facilitate long overlaps for annealing the gene. Yeast preferred codons were used where possible and Nco1 and Sal1 sites incorporated at the 5′ initiation codon and 3′ stop codon respectively.

The gene was assembled by kinasing each oligomer, pooling them in ligase buffer and then dissociating at 100°C and allowing to cool to room temperature. Ligase and ATP were added and the ligated gene was then cut with restriction enzymes Nco1 and sal1 purified by agarose gel electrophoresis.

The gene is designed to be expressed on the ADH2-GAPDH yeast regulatable promoter for internal expression (see above). Berghei 3 may also be expressed on the secretory pathway by cloning into pAB125, a vector containing the alpha factor-ADH2 leader/promoter sequence (see above).

Transformation of Yeast with CS Protein Analog Expression Vectors

Saccharomyces cerevisiae strain AB110 (Mat, leu2-04, or both Leu2-3 and leu2-112, pep3-4, his4-580, cir°) was transformed with the expression vectors (except for the Falc 4 construct) according to Hinnen et al., Proc Natl Acad Sci (USA) 75:1929-1933 (1978). Single-transformant colonies harboring ADH2/GAP-regulated vectors were grown in 2 ml of leu⁻ (leucine-depleted) selective media to late log or stationary phase. Only yeast harboring such vectors grow in this medium. Cultures were subsequently diluted 1:20 (v/v) in YEP (1% w/v yeast extract, 2% w/v peptone) with 1% glucose, and grown to saturation (about 36 hours) in this medium. Cells were lysed in the presence of sodium dodecyl sulfate (SDS) and dithiothreitol (DTT) and the lysates were clarified by centrifugation. Cleared lysates were subjected to polyacrylamide gel electrophoresis (Laemmli, U.K., Nature (1970) 277:680). Following staining with Coomassie blue, bands of appropriate size were observed in extracts from transformants containing the P. vivax plasmids and the P. falciparum plasmid. These bands were detected in those cells transformed with the respective expression vectors, while being absent from extracts of cells harboring control plasmids.

To confirm the identity of the bands, the proteins were also submitted to Western analysis. Cleared yeast lysates prepared as described above were electrophoresed on polyacrylamide gels (Laemmli, supra) and proteins were subsequently electroblotted onto nitrocellulose filters (Towbin et al., Proc Natl Acad Sci (USA) (1979) 76:3450). The filter was preincubated for 1 hour with 1% (BSA) in PBS and subsequently treated with antibodies to the native CS proteins for 12 hours at 4° C. The filters were washed with 1% BSA/PBS and a second goat anti-mouse antibody conjugated with horseradish peroxidase (Bio-Rad Laboratories, Richmond, California) added. Finally, the filters were incubated with horseradish peroxidase color development reagent (Bio-Rad, Richmond, California) and washed. The Western analysis showed that the proteins reacted with the antibodies.

CS Protein Analog Production in Yeast (Intracellular) and Purification

Transformants were grown in selective media (either Leu⁻ or Ura⁻) to increase plasmid copy number and in high glucose (8%) to keep the expression repressed.

The yeasts were then shifted to 2% glucose in YEP (a rich media for intracellular expression) for 48 hours (expression is seen as early as 24 hours).

Yeasts were separated from the media by filtration or centrifugation and the yeast broken, in an appropriate buffer. Protein purification was initiated by selective heat precipitation after dilution with an equal volume of water and the pH adjusted to 8.0 (100°C for 20 minutes; this not only serves as a purification step but also as a protease inhibition step). The precipitate was removed by filtration or centrifugation.

The CS protein analog in the supernatant was further purified by ion exchange chromatography on a cation exchanger by lowering the pH to 4.0 to allow binding. After washing the column at pH 5.0, a salt gradient is used to selectively remove the proteins including the CS protein analog. The analog is collected, concentrated and subjected to size exclusion chromatography.

Purified CS protein was concentrated and diafiltered. It was stored either frozen at -80°C or freeze-dried and stored moisture-free in a sealed vial.

CS Protein Analog Production in Yeast (Secretory) and Purification

Transformants were grown in selective media (either Leu⁻ or Ura⁻) to increase plasmid copy number and in high glucose (8%) to keep the expression repressed.

The yeast is then shifted to 2% glucose in a yeast secretion media (low protein) and grown for 90 hours (expression is seen as early as 36 hours, but 90 hours allows accumulation of CS protein analog in the media).

Yeast is removed from the media by filtration or centrifugation, the supernatant retained and adjusted to pH 8.0 (NaOH). Protein purification is initiated by selective heat precipitation (100°C for 20 minutes not only only serves as a purification step but also as a protease inhibition step). This precipitate is removed by filtration or centrifugation.

Purification of the analog from the supernatant was carried out as above.

Novel modifications of the above-described modes for carrying out the invention that are obvious to those of skill in that fields of molecular biology, bio chemistry, immunology, and related fields are intended to be within the scope of the invention.

## Claims

1. An immunogenic Plasmodium circumsporozoite (CS) protein analog that lacks one or more of the repeat epitopes of the native CS protein but has at least one nonrepeat flanking epitope from each nonrepeat flanking region of the native CS protein.

2. An immunogenic Plasmodium circumsporozoite (CS) protein analog comprising:

(a) an internal region composed of at least two iterations of a repeat sequence of a native Plasmodium CS protein, but fewer iterations of said sequence than are present in the native Plasmodium CS protein,

(b) an amino terminal region flanking the internal region having an amino acid sequence that defines at least one epitope of the nonrepeat amino terminal flanking region of the native Plasmodium CS protein; and

(c) a carboxy terminal region flanking the internal region having an amino acid sequence that defines at least one epitope of the nonrepeat carboxy terminal flanking region of the native Plasmodium CS protein.

3. The analog of claim 2 wherein the native plasmodium CS protein is P. vivax CS protein.

4. The analog of claim 3 wherein the internal region contains both types of repeat sequence of native P. vivax CS protein, there are 2 to 18 iterations of the repeat sequence, the amino terminal region contains at least amino acids 23-76 of native P. vivax CS protein and the carboxy terminal region contains at least amino acids 264-318 of native P. vivax CS protein.

5. The analogs of claim 3 wherein the internal region contains both types of repeat sequence of native P. vivax CS protein, there are 2 to 10 iterations of the repeat sequence, the amino terminal region consists of amino acids 23-91 of native P. vivax CS protein and the carboxy terminal region consists of amino acids 264-335 of the native P. vivax CS protein.

6. The analog of claim 3 wherein the analog has the amino acid sequence shown in Figure 1 or Figure 5.

7. The analog of claim 2 wherein the native Plasmodium CS protein is P. falciparum CS protein.

8. The analog of claim 7 wherein the internal region contains both types of repeat sequences of native P. falciparium CS protein, there are 2 to 40 iterations of the repeat sequence, the amino terminal flanking region contains at least amino acids 107-123 of native P. falciparum CS protein, and the carboxy terminal flanking region contains at least amino acids 289-356 of native P. falciparium CS protein.

9. The analog of claim 7 wherein the internal region contains both types of repeat sequences of native P. falciparium CS protein there are 2 to 20 iterations of the repeat sequence, the amino terminal region comprises amino acids 68-123 of native P. falciparum CS protein and the carboxy terminal region comprises amino acids 289-374 of the native P. falciparum CS protein.

10. The analog of claim 7 wherein the analog has the amino acid sequence shown in Figure 8 or Figure 10.

11. The analog of claim 2 wherein the native Plasmodium CS protein is P. berghei CS protein.

12. The analog of claim 11 wherein the internal region contains two types of repeat sequence of native P. berghei CS protein and there are 2 to 12 iterations of repeat sequence.

13. The analog of claim 11 wherein the analog has the amino acid sequence shown in Figure 11.

14. A DNA sequence encoding the analog of claims 2, 3, 4, 5, 6, 7, 8, 9, 10, 11, 12, or 13.

15. An expression vector for expressing a Plasmodium CS protein analog in a cell comprising the DNA of claim 14 and expression control sequences that are operably connected to said DNA and effective in directing expression of said DNA in said cell.

16. A recombinant cell containing the vector of claim 15 and being capable of producing the protein encoded by said DNA.

17. A method of producing a Plasmodium CS protein analog comprising growing the cell of claim 16 under conditions that permit the production of the protein encoded by the DNA.

18. A malaria vaccine composition comprising an immunogenic amount of the analog of claim 2, 3, 4, 5, 6, 7, 8, 9, 10, 11, 12 or 13 admixed with a pharmaceutically acceptable carrier.

# FIG. I

```
    MetGlyHisAsnValAspLeuCysLysAlaIleAsnLeuAsnGluValAspAlaSerSer
  3 ATGGGTCACAACGTAGATTTGTGTAAGGCTATCAACTTGAACGAAGTTGACGCTAGCTCT
    TACCCAGTGTTGCATCTAAACACATTCCGATAGTTGAACTTGCTTCAACTGCGATCGAGA
```

1 NCOI, 54 NHEI,

```
    LeuGlyAlaAlaHisValGlyGlnSerAlaSerArgGlyArgGlyLeuGlyGluAsnPro
 63 TTGGGTGCTGCTCACGTTGGTCAATCTGCTTCTAGAGGTAGAGGTTTGGGTGAAAACCCA
    AACCCACGACGAGTGCAACCAGTTAGACGAAGATCTCCATCTCCAAACCCACTTTTGGGT
```

93 XBAI,

```
    AspAspGluGluGlyAspAlaLysLysLysLysAspGlyLysLysAlaGluProLysAsn
123 GACGACGAAGAAGGTGACGCTAAGAAGAAGAAGGACGGTAAGAAGGCTGAACCAAAGAAC
    CTGCTGCTTCTTCCACTGCGATTCTTCTTCTTCCTGCCATTCTTCCGACTTGGTTTCTTG
```

```
    ProArgGluAsnLysLeuLysGlnProArgAspArgAlaAspGlyGlnProAlaGlyAsp
183 CCTCGAGAAAACAAGTTGAAGCAACCAAGAGACAGAGCTGACGGTCAACCAGCTGGTGAT
    GGAGCTCTTTTGTTCAACTTCGTTGGTTCTCTGTCTCGACTGCCAGTTGGTCGACCACTA
```

184 XHOI, 232 PVU2,

```
    ArgAlaAlaGlyGlnProAlaGlyAsnGlyAlaGlyGlyGlnAlaAlaGlyGlyAsnAla
243 AGGGCCGCTGGCCAGCCTGCCGGCAACGGTGCTGGTGGTCAAGCCGCGGGTGGTAACGCT
    TCCCGGCGACCGGTCGGACGGCCGTTGCCACGACCACCAGTTCGGCGCCCACCATTGCGA
```

251 BALI, 261 NAEI, 286 SAC2,

```
    GlyGlyGlyGlnGlyGlnAsnAsnGluGlyAlaAsnAlaProAsnAlaLysSerValLys
303 GGTGGTGGTCAAGGTCAAAACAACGAAGGTGCTAACGCTCCAAACGCTAAGTCTGTTAAG
    CCACCACCAGTTCCAGTTTTGTTGCTTCCACGATTGCGAGGTTTGCGATTCAGACAATTC
```

```
    GluTyrLeuAspLysLeuGluThrThrValGlyThrGluTrpThrProCysSerValThr
363 GAATACTTGGACAAGTTGGAAACCACCGTTGGTACCGAATGGACCCCATGTTCTGTTACC
    CTTATGAACCTGTTCAACCTTTGGTGGCAACCATGGCTTACCTGGGGTACAAGACAATGG
```

384 HGIE2, 393 KPNI,

```
    CysGlyValGlyValArgValArgSerArgValAsnAlaAlaAsnLysLysProGluAsp
423 TGTGGTGTTGGTGTTAGAGTTAGATCCAGAGTTAACGCTGCTAACAAGAAGCCAGAAGAT
    ACACCACAACCACAATCTCAATCTAGGTCTCAATTGCGACGATTGTTCTTCGGTCTTCTA
```

453 HPAI, 479 BGL2,

```
    LeuAM Ser
483 CTATAGTCGAC
    GATATCAGCTG
```

488 SALI,

Synthetic Oligonucleotides For Vivax3

```
         5'                                                    3'
 1-27   CATGGGTCACAACGTAGATTTGTGTAA
 2-46   GGCTATCAACTTGAACGAAGTTGACGCTAGCTCTTTGGGTGCTGCT
 3-46   CACGTTGGTCAATCTGCTTCTAGAGGTAGAGGTTTGGGTGAAAACC
 4-46   CAGACGACGAAGAAGGTGACGCTAAGAAGAAGAAGGACGGTAAGAA
 5-46   GGCTGAACCAAAGAACCCTCGAGAAAACAAGTTGAAGCAACCAAGA
 6-46   GACAGAGCTGACGGTCAACCAGCTGGTGATAGGGCCGCTGGCCAGC
 7-46   CTGCCGGCAACGGTGCTGGTGGTCAAGCCGCGGGTGGTAACGCTGG
 8-46   TGGTGGTCAAGGTCAAAACAACGAAGGTGCTAACGCTCCAAACGCT
 9-46   AAGTCTGTTAAGGAATACTTGGACAAGTTGGAAACCACCGTTGGTA
10-46   CCGAATGGACCCCATGTTCTGTTACCTGTGGTGTTGGTGTTAGAGT
11-46   TAGATCCAGAGTTAACGCTGCTAACAAGAAGCCAGAAGATCTATAG
12*46   CAACTTCGTTCAAGTTGATAGCCTTACACAAATCTACGTTGTGACC
13*46   CTAGAAGCAGATTGACCAACGTGAGCAGCACCCAAAGAGCTAGCGT
14*46   AGCGTCACCTTCTTCGTCGTCTGGGTTTTCACCCAAACCTCTACCT
15*46   CTCGAGGGTTCTTTGGTTCAGCCTTCTTACCGTCCTTCTTCTTCTT
16*46   GCTGGTTGACCGTCAGCTCTGTCTCTTGGTTGCTTCAACTTGTTTT
17*46   ACCACCAGCACCGTTGCCGGCAGGCTGGCCAGCGGCCCTATCACCA
18*46   CGTTGTTTTGACCTTGACCACCACCAGCGTTACCACCCGCGGCTTG
19*46   TCCAAGTATTCCTTAACAGACTTAGCGTTTGGAGCGTTAGCACCTT
20*46   AACAGAACATGGGGTCCATTCGGTACCAACGGTGGTTTCCAACTTG
21*46   TAGCAGCGTTAACTCTGGATCTAACTCTAACACCAACACCACAGGT
22*27   TCGACTATAGATCTTCTGGCTTCTTGT
```

Vivax 3

```
      1-27   2-46   3-46   4-46   5-46   6-46   7-46   8-46   9-46   10-46  11-46
Nco1 ===============================================================================  Sal1
      12*46  13*46  14*46  15*46  16*46  17*46  18*46  19*46  20*46  21*46  22*27
```

# FIG. 2

| BAM: | 0 |
| XBA: | 129 |
| XBA: | 191 |
| SAC: | 545 |
| NCO: | 1160 |
| BGLII: | 1186 |
| PVUII: | 1224 |
| SAL: | 1991 |
| H3: | 2133 |
| SACII: | 2529 |
| BAM: | 2922 |
| PST: | 5911 |

| | ADH2GAPp: | 0 | 1169 |
| | GH ENV: | 1169 | 1987 |
| | GAPt: | 1987 | 2922 |
| | pBR322(H3-SAL): | 2922 | 6663 |

FIG. 3

FIG. 4

# FIG. 5

```
         23                                                              41
      MetGlyHisAsnValAspLeuCysLysAlaIleAsnLeuAsnGluValAspAlaSerSer
   3  ATGGGTCACAACGTAGATTTGTGTAAGGCTATCAACTTGAACGAAGTTGACGCTAGCTCT
      TACCCAGTGTTGCATCTAAACACATTCCGATAGTTGAACTTGCTTCAACTGCGATCGAGA

      1 NCOI, 54 NHEI,
                                                                        61
       LeuGlyAlaAlaHisValGlyGlnSerAlaSerArgGlyArgGlyLeuGlyGluAsnPro
   63 TTGGGTGCTGCTCACGTTGGTCAATCTGCTTCTAGAGGTAGAGGTTTGGGTGAAAACCCA
      AACCCACGACGAGTGCAACCAGTTAGACGAAGATCTCCATCTCCAAACCCACTTTTGGGT

      93 XBAI,
                                                                        81
      AspAspGluGluGlyAspAlaLysLysLysLysAspGlyLysLysAlaGluProLysAsn
  123 GACGACGAAGAAGGTGACGCTAAGAAGAAGAAGGACGGTAAGAAGGCTGAACCAAAGAAC
      CTGCTGCTTCTTCCACTGCGATTCTTCTTCTTCCTGCCATTCTTCCGACTTGGTTTCTTG

      ProArgGluAsnLysLeuLysGlnProGlyAspArgAlaAspGlyGlnProAlaGlyAsp
  183 CCTCGAGAAAACAAGTTGAAGCAACCAGGAGACAGAGCTGACGGTCAACCAGCTGGTGAT
      GGAGCTCTTTTGTTCAACTTCGTTGGTCCTCTGTCTCGACTGCCAGTTGGTCGACCACTA

      184 XHOI, 232 PVU2,
                                     264                        275
      ArgAlaAlaGlyGlnProAlaGlyAsnGlyAlaGlyGlyGlnAlaAlaGlyGlyAsnAla
  243 AGGGCCGCTGGCCAGCCTGCCGGCAACGGTGCTGGTGGTCAAGCCGCGGGTGGTAACGCT
      TCCCGGCGACCGGTCGGACGGCCGTTGCCACGACCACCAGTTCGGCGCCCACCATTGCGA

      251 BALI, 261 NAEI, 286 SAC2,
                                                                       295
      GlyGlyGlnGlyGlnAsnAsnGluGlyAlaAsnAlaProAsnGluLysSerValLysGlu
  303 GGTGGTCAAGGTCAAAACAACGAAGGTGCTAACGCTCCAAACGAGAAGTCTGTTAAGGAA
      CCACCAGTTCCAGTTTTGTTGCTTCCACGATTGCGAGGTTTGCTCTTCAGACAATTCCTT
                                                                       315
      TyrLeuAspLysValArgAlaThrValGlyThrGluTrpThrProCysSerValThrCys
  363 TACTTGGACAAGGTTAGAGCTACCGTTGGTACCGAATGGACCCCATGTTCTGTTACCTGT
      ATGAACCTGTTCCAATCTCGATGGCAACCATGGCTTACCTGGGGTACAAGACAATGGACA

      390 KPNI,
                                                                       335
      GlyValGlyValArgValArgSerArgValAsnAlaAlaAsnLysLysProGluAspLeu
  423 GGTGTTGGTGTTAGAGTTAGATCCAGAGTTAACGCTGCTAACAAGAAGCCAGAAGATCTA
      CCACAACCACAATCTCAATCTAGGTCTCAATTGCGACGATTGTTCTTCGGTCTTCTAGAT

      450 HPAI, 476 BGL2,

      AM Ser
  483 TAGTCGAC
      ATCAGCTG

      485 SALI,
```

Synthetic Oligonucleotides For Vivax3.1

```
        5'                                    3'
  5-28  TCGAGAAAACAAGTTGAAGCAACCAGGA
  8-43  TGGTCAAGGTCAAAACAACGAAGGTGCTAACGCTCCAAACGAG
  9-43  AAGTCTGTTAAGGAATACTTGGACAAGGTTAGAGCTACCGTTG
 16*47  GCTGGTTGACCGTCAGCTCTGTCTCCTGGTTGCTTCAACTTGTTTTC
 18*43  CGTTGTTTTGACCTTGACCACCAGCGTTACCACCCGCGGCTTG
 19*46  TCCAAGTATTCCTTAACAGACTTCTCGTTTGGAGCGTTAGCACCTT
 20*24  GTACCAACGGTAGCTCTAACCTTG
```

```
                6-46    7-46
              ┌───────────────→
       5-28 \  ┌17*46, ┌8-43    9-43
Xho1       →  ╲─────╱ ──────────────   Asp718   Vivax 3.1 Correction
       16*47 V  18*43  19*46  20*24
```

# FIG. 6

Sacl 398

BamH1 0

Pstl 1046

Xbal 1266

Pstl 12502

ADH2GAPp

ALPHAFACTOR1

pBR322

*pAB125*

ALPHAFACTORt

BamH1 9544
EcoR1 9538

Sall 9266

8KbLambda

| BamH1: | 0 |
| Sacl: | 398 |
| Pstl: | 1046 |
| Xbal: | 1266 |
| Sall: | 9266 |
| EcoR1: | 9538 |
| BamH1: | 9544 |
| Pstl: | 12502 |

| | ADH2GAPp: | 0 | 1046 |
| | ALPHAFACTOR1: | 1046 | 1266 |
| | 8KbLambda: | 1266 | 9266 |
| | ALPHAFACTORt: | 9266 | 9544 |
| | pBR322: | 9544 | 13370 |

FIG. 7

# FIG. 8

```
                                                                    61
    43
    MetAlaGlyThrAsnLeuTyrAsnGluLeuGluMetAsnTyrTyrGlyLysGlnGluAsn
  3 ATGGCTGGTACCAACTTGTACAACGAATTGGAAATGAACTACTACGGTAAGCAAGAAAAC
    TACCGACCATGGTTGAACATGTTGCTTAACCTTTACTTGATGATGCCATTCGTTCTTTTG

  1 NCOI, 9 KPNI,
                                                                    81

    TrpTyrSerLeuLysLysAsnSerArgSerLeuGlyGluAsnAspAspAlaAsnAsnAsn
 63 TGGTACTCTTTGAAGAAGAACTCTAGGTCTTTGGGTGAAAACGATGACGCTAACAACAAC
    ACCATGAGAAACTTCTTCTTGAGATCCAGAAACCCACTTTTGCTACTGCGATTGTTGTTG
                                                                   101
    AsnGlyAspAsnGlyArgGluGlyLysAspGluAspLysArgAspGlyAsnAsnGluAsp
123 AACGGTGACAACGGTAGAGAAGGTAAGGACGAAGACAAGAGAGACGGTAACAACGAAGAC
    TTGCCACTGTTGCCATCTCTTCCATTCCTGCTTCTGTTCTCTCTGCCATTGTTGCTTCTG
                                                                   121
    AsnGluLysLeuArgLysProLysHisLysLysLeuLysGlnProGlyAspGlyAsnPro
183 AACGAAAAGTTGAGAAAGCCAAAGCACAAGAAGCTTAAGCAACCAGGTGACGGTAACCCA
    TTGCTTTTCAACTCTTTCGGTTTCGTGTTCTTCGAATTCGTTGGTCCACTGCCATTGGGT

213 HIND3, 216 AFL2, 234 BSTE2,
                                                                289290
    AspProAsnAlaAsnProAsnValAspProAsnAlaAsnProAsnAlaAsnProAsnLys
243 GACCCAAACGCTAACCCAAACGTGGATCCTAACGCCAACCCTAATGCTAATCCAAACAAG
    CTGGGTTTGCGATTGGGTTTGCACCTAGGATTGCGGTTGGGATTACGATTAGGTTTGTTC

266 BAMHI,
                                                                   310
    AsnAsnGlnGlyAsnGlyGlnGlyHisAsnMetProAsnAspProAsnArgAsnValAsp
303 AACAACCAAGGTAACGGTCAAGGTCACAACATGCCAAACGACCCAAACAGAAACGTTGAC
    TTGTTGGTTCCATTGCCAGTTCCAGTGTTGTACGGTTTGCTGGGTTTGTCTTTGCAACTG
                                                                   330
    GluAsnAlaAsnAlaAsnAsnAlaValLysAsnAsnAsnAsnGluGluProSerAspLys
363 GAGAACGCTAACGCTAACAACGCTGTTAAGAACAACAACAACGAAGAACCATCTGACAAG
    CTCTTGCGATTGCGATTGTTGCGACAATTCTTGTTGTTGTTGCTTCTTGGTAGACTGTTC
                                                                   350
    HisIleGluGlnTyrLeuLysLysIleLysAsnSerIleSerThrGluTrpSerProCys
423 CACATCGAACAATACTTGAAGAAGATCAAGAACTCTATCTCTACCGAATGGTCTCCATGT
    GTGTAGCTTGTTATGAACTTCTTCTAGTTCTTGAGATAGAGATGGCTTACCAGAGGTACA
                                                                   370
    SerValThrCysGlyAsnGlyIleGlnValArgIleLysProGlySerAlaAsnLysPro
483 TCTGTTACCTGTGGTAACGGTATCCAAGTTAGAATCAAGCCAGGTTCTGCTAACAAGCCT
    AGACAATGGACACCATTGCCATAGGTTCAATCTTAGTTCGGTCCAAGACGATTGTTCGGA

523 ALWN1, 540 MST2,
                  374
    LysAspGluLeuAM Ser
543 AAGGACGAATTGTAGTCGAC
    TTCCTGCTTAACATCAGCTG

557 SALI,
```

Synthetic Oligonucleotides For Falc3

```
            5'                                                              3'
 1-28   CATGGCTGGTACCAACTTGTACAACGAA
 2-48   TTGGAAATGAACTACTACGGTAAGCAAGAAAACTGGTACTCTTTGAAG
 3-48   AAGAACTCTAGGTCTTTGGGTGAAAACGATGACGCTAACAACAACAAC
 4-48   GGTGACAACGGTAGAGAAGGTAAGGACGAAGACAAGAGAGACGGTAAC
 5-48   AACGAAGACAACGAAAAGTTGAGAAAGCCAAAGCACAAGAAGCTTAAG
 6-48   CAACCAGGTGACGGTAACCCAGACCCAAACGCTAACCCAAACGTGGAT
 7-48   CCTAACGCCAACCCTAATGCTAATCCAAACAAGAACAACCAAGGTAAC
 8-48   GGTCAAGGTCACAACATGCCAAACGACCCAAACAGAAACGTTGACGAG
 9-48   AACGCTAACGCTAACAACGCTGTTAAGAACAACAACAACGAAGAACCA
10-48   TCTGACAAGCACATCGAACAATACTTGAAGAAGATCAAGAACTCTATC
11-48   TCTACCGAATGGTCTCCATGTTCTGTTACCTGTGGTAACGGTATCCAA
12-48   GTTAGAATCAAGCCAGGTTCTGCTAACAAGCCTAAGGACGAATTGTAG
13*48   CTTACCGTAGTAGTTCATTTCCAATTCGTTGTACAAGTTGGTACCAGC
14*48   TTCACCCAAAGACCTAGAGTTCTTCTTCAAAGAGTACCAGTTTTCTTG
15*48   CTTACCTTCTCTACCGTTGTCACCGTTGTTGTTGTTAGCGTCATCGTT
16*48   TCTCAACTTTTCGTTGTCTTCGTTGTTACCGTCTCTCTTGTCTTCGTC
17*48   GTCTGGGTTACCGTCACCTGGTTGCTTAAGCTTCTTGTGCTTTGGCTT
18*48   ATTAGCATTAGGGTTGGCGTTAGGATCCACGTTTGGGTTAGCGTTTGG
19*48   GTTTGGCATGTTGTGACCTTGACCGTTACCTTGGTTGTTCTTGTTTGG
20*48   AACAGCGTTGTTAGCGTTAGCGTTCTCGTCAACGTTTCTGTTTGGGTC
21*48   GTATTGTTCGATGTGCTTGTCAGATGGTTCTTCGTTGTTGTTGTTCTT
22*48   AGAACATGGAGACCATTCGGTAGAGATAGAGTTCTTGATCTTCTTCAA
23*48   AGCAGAACCTGGCTTGATTCTAACTTGGATACCGTTACCACAGGTAAC
24*28   TCGACTACAATTCGTCCTTAGGCTTGTT
```

Falc 3

```
         1-28   2-48    3-48    4-48    5-48    6-48    7-48    8-48    9-48    10-48   11-48   12-48
Ncol ═══════════════════════════════════════════════════════════════════════════════════════════ Sal1
         13*48   14*48   15*48   16*48   17*48   18*48   19*48   20*48   21*48   22*48   23*48   24*28
```

FIG. 9

## FIG. IO

```
                        ↓68                                           83
    LeuAspLysArgAsnSerArgSerLeuGlyGluAsnAspAspAlaAsnAsnAsnAsnGly
  2 CTAGATAAAAGAAACTCTAGGTCTTTGGGTGAAAACGATGACGCTAACAACAACAACGGT
    GATCTATTTTCTTTGAGATCCAGAAACCCACTTTTGCTACTGCGATTGTTGTTGTTGCCA

  1 XBAI,                                                       103

    AspAsnGlyArgGluGlyLysAspGluAspLysArgAspGlyAsnAsnGluAspAsnGlu
 62 GACAACGGTAGAGAAGGTAAGGACGAAGACAAGAGAGACGGTAACAACGAAGACAACGAA
    CTGTTGCCATCTCTTCCATTCCTGCTTCTGTTCTCTCTGCCATTGTTGCTTCTGTTGCTT
                                                                123
    LysLeuArgLysProLysHisLysLysLeuLysGlnProGlyAspGlyAsnProAspPro
122 AAGTTGAGAAAGCCAAAGCACAAGAAGCTTAAGCAACCAGGTGACGGTAACCCAGACCCA
    TTCAACTCTTTCGGTTTCGTGTTCTTCGAATTCGTTGGTCCACTGCCATTGGGTCTGGGT

146 HIND3, 149 AFL2, 167 BSTE2,                        289      292

    AsnAlaAsnProAsnValAspProAsnAlaAsnProAsnAlaAsnProAsnLysAsnAsn
182 AACGCTAACCCAAACGTGGATCCTAACGCCAACCCTAATGCTAATCCAAACAAGAACAAC
    TTGCGATTGGGTTTGCACCTAGGATTGCGGTTGGGATTACGATTAGGTTTGTTCTTGTTG

199 BAMHI,                                                     312

    GlnGlyAsnGlyGlnGlyHisAsnMetProAsnAspProAsnArgAsnValAspGluAsn
242 CAAGGTAACGGTCAAGGTCACAACATGCCAAACGACCCAAACAGAAACGTTGACGAGAAC
    GTTCCATTGCCAGTTCCAGTGTTGTACGGTTTGCTGGGTTTGTCTTTGCAACTGCTCTTG
                                                                332
    AlaAsnAlaAsnAsnAlaValLysAsnAsnAsnAsnGluGluProSerAspLysHisIle
302 GCTAACGCTAACAACGCTGTTAAGAACAACAACAACGAAGAACCATCTGACAAGCACATC
    CGATTGCGATTGTTGCGACAATTCTTGTTGTTGTTGCTTCTTGGTAGACTGTTCGTGTAG
                                                                352
    GluGlnTyrLeuLysLysIleLysAsnSerIleSerThrGluTrpSerProCysSerVal
362 GAACAATACTTGAAGAAGATCAAGAACTCTATCTCTACCGAATGGTCTCCATGTTCTGTT
    CTTGTTATGAACTTCTTCTAGTTCTTGAGATAGAGATGGCTTACCAGAGGTACAAGACAA
                                                                372
    ThrCysGlyAsnGlyIleGlnValArgIleLysProGlySerAlaAsnLysProLysAsp
422 ACCTGTGGTAACGGTATCCAAGTTAGAATCAAGCCAGGTTCTGCTAACAAGCCTAAGGAC
    TGGACACCATTGCCATAGGTTCAATCTTAGTTCGGTCCAAGACGATTGTTCGGATTCCTG

456 ALWN1, 473 MST2,                                           392

    GluLeuAspTyrGluAsnAspAsnGluLysLysIleCysLysMetGluLysCysSerSer
482 GAATTGGACTACGAAAACGACAACGAAAAGAAGATTTGTAAGATGGAAAAGTGTTCTTCC
    CTTAACCTGATGCTTTTGCTGTTGCTTTTCTTCTAAACATTCTACCTTTTCACAAGAAGG

    AM Ser
542 TAGTCGAC
    ATCAGCTG

544 SALI,


602
```

# FIG. II

```
         MetGlyGlnAsnLysIleIleGlnAlaGlnArgAsnLeuAsnGluLeuCysTyrAsnGlu
    3    ATGGGTCAAAACAAGATTATCCAAGCCCAAAGAAACTTGAACGAATTGTGTTACAACGAA
         TACCCAGTTTTGTTCTAATAGGTTCGGGTTTCTTTGAACTTGCTTAACACAATGTTGCTT

    1 NCOI,


         GlyAsnAspAsnLysLeuTyrHisValLeuAsnSerLysAsnGlyLysIleTyrAsnArg
   63    GGTAACGACAACAAGTTGTACCACGTCTTGAACTCTAAGAACGGTAAGATTTACAACAGA
         CCATTGCTGTTGTTCAACATGGTGCAGAACTTGAGATTCTTGCCATTCTAAATGTTGTCT


         AsnThrValAsnArgLeuLeuAlaAspAlaProGluGlyLysLysAsnGluLysLysAsn
  123    AACACTGTCAACAGATTGTTGGCCGACGCTCCAGAAGGTAAGAAGAACGAAAAGAAGAAC
         TTGTGACAGTTGTCTAACAACCGGCTGCGAGGTCTTCCATTCTTCTTGCTTTTCTTCTTG


         GluLysIleGluArgAsnAsnLysLeuLysGlnProProProProProAsnProAsnAsp
  183    GAAAAGATTGAAAGAAACAACAAGTTGAAGCAACCACCACCACCCCCCAACCCAAACGAC
         CTTTTCTAACTTTCTTTGTTGTTCAACTTCGTTGGTGGTGGTGGGGGGTTGGGTTTGCTG


         ProAlaProProAsnAlaAsnAspGlyGlyAsnAsnAsnAsnLysAsnAsnAsnAsnAsp
  243    CCAGCTCCACCAAACGCTAACGACGGTGGTAACAACAATAATAAGAACAACAATAATGAC
         GGTCGAGGTGGTTTGCGATTGCTGCCACCATTGTTGTTATTATTCTTGTTGTTATTACTG


         AspSerTyrIleProSerAlaGluLysIleLeuGluPheValLysGlnIleArgAspSer
  303    GACTCTTACATCCCATCTGCTGAAAAGATCTTGGAATTCGTTAAGCAAATCAGAGACTCT
         CTGAGAATGTAGGGTAGACGACTTTTCTAGAACCTTAAGCAATTCGTTTAGTCTCTGAGA

  328 BGL2, 336 ECORI,


         IleThrGluGluTrpSerGlnCysAsnValThrCysGlySerGlyIleArgValArgLys
  363    ATTACCGAAGAATGGTCCCAATGTAACGTCACCTGTGGTTCTGGTATCAGAGTTAGAAAG
         TAATGGCTTCTTACCAGGGTTACATTGCAGTGGACACCAAGACCATAGTCTCAATCTTTC


         ArgLysGlySerAsnLysLysAlaGluAspLeuThrLeuGluAspIleAspThrGluIle
  423    AGAAAGGGTTCTAACAAGAAGGCCGAAGACTTGACTTTGGAAGACATCGACACTGAAATC
         TCTTTCCCAAGATTGTTCTTCCGGCTTCTGAACTGAAACCTTCTGTAGCTGTGACTTTAG

  424 XMNI,


         CysLysMetAspLysCysSerSerAM
  483    TGTAAGATGGACAAGTGTTCTTCTTAGTCGAC
         ACATTCTACCTGTTCACAAGAAGAATCAGCTG

  509 SALI,
```

EP 0 392 820 A2

## FIG. 12

Berghei 3

```
    MetGlyGlnAsnLysIleIleGlnAlaGlnArgAsnLeuAsnGluLeuCysTyrAsnGluGlyAsnAspAsnLys      25
5'       1-28                    3'5'            2-48                            3'
    CATGGGTCAAAACAAGATTATCCAAGCCCAAAGAAACTTGAACGAATTGTGTTACAACGAAGGTAACGACAACAAG
       CCAGTTTTGTTCTAATAGGTTCGGGTTTCTTTGAACTTGCTTAACACAATGTTGCTTCCATTGCTGTTGTTC
Ncol                          12*48                            5'3'            13*48

    LeuTyrHisValLeuAsnSerLysAsnGlyLysIleTyrAsnArgAsnThrValAsnArgLeuLeuAlaAspAla      50
5'           3-48                               3'5'            4-48
    TTGTACCACGTCTTGAACTCTAAGAACGGTAAGATTTACAACAGAAACACTGTCAACAGATTGTTGGCCGACGCT
    AACATGGTGCAGAACTTGAGATTCTTGCCATTCTAAATGTTGTCTTTGTGACAGTTGTCTAACAACCGGCTGCGA
                         5'3'                   14*48                        5'3'

    ProGluGlyLysLysAsnGluLysLysAsnGluLysIleGluArgAsnAsnLysLeuLysGlnPro|ProProPro      75
                 3'5'                  5-48                            3'5'
    CCAGAAGGTAAGAAGAACGAAAAGAAGAACGAAAAGATTGAAAGAAACAACAAGTTGAAGCAACCA CCACCACCC
    GGTCTTCCATTCTTCTTGCTTTTCTTCTTGCTTTTCTAACTTTCTTTGTTGTTCAACTTCGTTGGT GGTGGTGGG
      15*48                                5'3'                        16*48

    ProAsnProAsnAsp|ProAlaProProAsnAlaAsnAsp|GlyGlyAsnAsnAsnAsnLysAsnAsnAsnAsnAsp    100
      6-48                                    3'5'            7-48
    CCCAACCCAAACGAC CCAGCTCCACCCAACGCTAACGAC GGTGGTAACAACAATAATAAGAACAACAATAATGAC
    GGGTTGGGTTTGCTG GGTCGAGGTGGGTTGCGATTGCTG CCACCATTGTTGTTATTATTCTTGTTGTTATTACTG
                5'3'                        17*48                        5'3'

    AspSerTyrIleProSerAlaGluLysIleLeuGluPheValLysGlnIleArgAspSerIleThrGluGluTrp     125
             3'5'                  8-48                            3'5'
    GACTCTTACATCCCATCTGCTGAAAAGATCTTGGAATTCGTTAAGCAAATCAGAGACTCTATTACCGAAGAATGG
    CTGAGAATGTAGGGTAGACGACTTTTCTAGAACCTTAAGCAATTCGTTAGTCTCTGAGATAATGGCTTCTTACC
      18*48                    Bgl 2       EcoRI 5'3'                    19*48

    SerGlnCysAsnValThrCysGlySerGlyIleArgValArgLysArgLysGlySerAsnLysLysAlaGluAsp     150
      9-48                               3'5'            10-48
    TCCCAATGTAACGTCACCTGTGGTTCTGGTATCAGAGTTAGAAAGAGAAAGGGTTCTAACAAGAAGGCCGAAGAC
    AGGGTTACATTGCAGTGGACACCAAGACCATAGTCTCAATCTTTCTCTTTCCCAAGATTGTTCTTCCGGCTTCTG
                5'3'                    20*48                        5'3'

    LeuThrLeuGluAspIleAspThrGluIleCysLysMetAspLysCysSerSerSTOP                       168
             3'5'                  11-48                    3'
    TTGACTTTGGAAGACATCGACACTGAAATCTGTAAGATGGACAAGTGTTCTTCTTAG
    AACTGAAACCTTCTGTAGCTGTGACTTTAGACATTCTACCTGTTCACAAGAAGAATCAGCT
      21*48                        5'3'                22*28      Sal I 5'
```

```
            P 1   P 2   P 3   P 4   P 5   P 6   P 7   P 8   P 9   P 10   P 11
20x48 ├─────────┼─────┼─────┼─────┼─────┼─────┼─────┼─────┼─────┼──────┼────────
2x28     12  P  13  P  14  P  15  P  16  P  17  P  18  P  19  P  20  P  21  P  22  P
```